# EUROPEAN PATENT SPECIFICATION

(11) **EP 1 620 068 B1**
(45) Date of publication and mention of the grant of the patent: **09.05.2012**
(21) Application number: 04725382.8
(22) Date of filing: 02.04.2004
(51) Int. Cl.: A61K 8/58, A61Q 1/02, A61Q 1/06, A61Q 1/14, A61Q 19/00

(54) **COSMETIC COMPOSITION COMPRISING A VOLATILE FATTY PHASE**
KOSMETISCHE ZUSAMMENSETZUNG ENTHALTEND EINE FLÜCHTIGE FETTPHASE
COMPOSITION COSMETIQUE COMPRENANT UNE PHASE LIPIDIQUE VOLATILE

(30) Priority: 04.04.2003 FR 0304259; 10.04.2003 US 461400 P; 20.05.2003 FR 0306068; 23.05.2003 US 443793
(43) Date of publication of application: 01.02.2006
(60) Divisional application: 11175076.6
(73) Proprietor: L'Oréal, 75008 Paris (FR)
(72) Inventor: AUGUSTE, Frédéric, F-94550 Chevilly-Larue (FR)
(74) Representative: Boulard, Denis
(86) International application number: PCT/EP2004/004523
(87) International publication number: WO 2004/087077

(56) References cited:
- EP-A- 0 333 432
- EP-A- 1 043 015
- EP-A1- 0 610 014
- EP-A1- 0 862 913
- WO-A-01/00141
- WO-A-96/06594
- WO-A1-03/042221
- WO-A2-03/013447
- DE-A- 4 341 652
- US-B1- 6 338 839
- ANONYMOUS: "Dow Corning 200 Fluid, 2 cSt and 5 cSt", DOW CORNING CORPORATION MATERIAL SAFETY DATA SHEET,, [Online] 1 April 2006 (2006-04-01), pages 1-8, XP002520874, Retrieved from the Internet: URL:http://resource.invensys.com/instrumen tation/msds/pdf/msds_031_d.pdf> [retrieved on 2009-03-25]
- ANONYMOUS: 'Certificate of Chemical Equivalency' INTERNET CITATION, [Online] 11 January 2010, page 1 Retrieved from the Internet: <URL:https://www.xiameter.com/en/Pages/Chem icalEquivalency.aspx?ChemicalID=04088401> [retrieved on 2010-10-27]
- ANONYMOUS: 'XIAMETER(R) RBG-0910 GUM' INTERNET CITATION, [Online] 13 October 2010, pages 1 - 7 XIAMETER(R) Material Safety Data Sheet Retrieved from the Internet: <URL:https://www.xiameter.com/EN/Pages/Retr ieveDocument.aspx?type=SDS&DocumentId=09000 7b2815aa75e&s=39467> [retrieved on 2010-10-27]
- ANONYMOUS: 'DOW CORNING(R) 2-1184 FLUID' INTERNET CITATION, [Online] 22 October 2010, pages 1 - 9 DOW CORNING CORPORATION Material Safety Data Sheet Retrieved from the Internet: <URL:http://www3.dowcorning.com/DataFiles/0 90007b2815c18c7.pdf> [retrieved on 2010-10-27]

## Description

The present invention relates to a cosmetic composition comprising a volatile silicone oil. The invention also relates to a non-therapeutic care or make-up process for human keratin materials, comprising the application of the composition to the keratin materials.

Volatile silicone oils are commonly used in cosmetic compositions for their good cosmetic properties, especially their pleasant feel on contact with the skin. These oils also evaporate quickly after they have been applied to keratin materials. However, their rate of evaporation should not be too high, so as to allow the user sufficient time to apply the cosmetic product to the keratin materials, especially uniformly.

The volatile silicone oils most commonly used in cosmetic products are cyclic silicones containing from 4 to 6 siloxane groups (generally known as D4, D5 and D6) and containing only methyl groups.WO 03/013447 A2 discloses the use of D5 as volatile silicone oil. It also discloses the use of a combination of volatile silicone oils comprising dodecamethylpentasiloxane, decamethyltetrasiloxane and at least 30% octamethyltrisiloxane.

The aim of the present invention is thus to provide an alternative for formulating compositions comprising volatile oils, and especially volatile oils that are compatible with the ingredients usually used in cosmetic compositions.

The inventors have discovered that such a composition is obtained by using at least two particular volatile silicone oils that give the volatile fatty phase a particular evaporation profile.

More specifically, one subject of the invention is a composition comprising, in a physiologically acceptable medium, a volatile silicone fatty phase comprising at least one non-cyclic volatile silicone oil and from 0 to 5 % by weight of cyclic volatile silicone oil, relative to the total weight of the volatile silicone fatty phase, the volatile silicone fatty phase having an evaporation profile such that the mass of volatile silicone oil evaporated after 30 minutes is from 2 mg/cm² to 9 mg/cm², the volatile silicon fatty phase comprises a mixture of dodecamethylpentasiloxane and decamethyltetrasiloxane, wherein the evaporation profile is determined according to protocol 1 described below.

A subject of the disclosure is also a composition comprising, in a physiologically acceptable medium, a volatile fatty phase comprising at least one non-cyclic volatile oil with a surface tension of less than 21 mN/m, preferably of less than 20 mN/m, and from 0 to 5 % by weight of cyclic volatile silicone oil, relative to the total weight of the volatile silicone fatty phase, the volatile fatty phase having an evaporation profile such that the mass of non-cyclic volatile oil evaporated after 30 minutes ranges from 2 mg/cm² to 9 mg/cm².

A subject of the invention is also a cosmetic make-up process or non-therapeutic treatment process for keratin materials, comprising the application to the keratin materials of a composition of the invention as defined above.

The term "volatile oil" means an oil (or a non-aqueous medium) that may evaporate on contact with the skin in less than one hour at room temperature and atmospheric pressure. The volatile oil is a volatile cosmetic oil, which is liquid at room temperature, especially having a non-zero vapour pressure, at room temperature and atmospheric pressure, in particular having a vapour pressure ranging from 0.13 Pa to 40 000 Pa (10⁻³ to 300 mmHg), preferably ranging from 1.3 Pa to 13 000 Pa (0.01 to 100 mmHg) and preferentially ranging from 1.3 Pa to 1300 Pa (0.01 to 10 mmHg).

The composition according to the invention comprises a physiologically acceptable medium, especially a cosmetically or dermatologically acceptable medium, i.e. a medium that is compatible with keratin materials such as the skin, mucous membranes, the hair, the eyelashes, the eyebrows and the nails.

The composition according to the invention contains a volatile silicone fatty phase comprising at least one non-cyclic volatile silicone oil and from 0 to 5 % by weight of cyclic volatile silicone oil, relative to the total weight of the volatile silicone fatty phase, the volatile silicone fatty phase having an evaporation profile such that the mass of volatile silicone oil evaporated after 30 minutes ranges from 2 mg/cm² to 9 mg/cm², preferably ranges from 2 mg/cm² to 8 mg/cm², preferentially ranges from 2 mg/cm² to 7 mg/cm², more preferentially ranges from 2.3 mg/cm² to 6 mg/cm² and even more preferentially ranges from 3 mg/cm² to 5.5 mg/cm², and especially ranges from 3 mg/cm² to 4.7 mg/cm².

The evaporation rate of the oil is measured according to the protocol described below.

Moreover, according to the present disclosure , the composition can contain a volatile fatty phase comprising at least one non-cyclic volatile oil with a surface tension of less than 21 mN/m, preferably of less than 20 mN/m, and from 0 to 5 % by weight of cyclic volatile silicone oil, relative to the total weight of the volatile silicone fatty phase, the volatile fatty phase having an evaporation profile such that the mass of volatile oil evaporated after 30 minutes ranges from 2 mg/cm² to 9 mg/cm², preferably from 2 mg/cm² to 8 mg/cm², preferentially ranges from 2 mg/cm² to 7 mg/cm², more preferentially ranges from 2.3 mg/cm² to 6 mg/cm² and even more preferentially ranges from 3 mg/cm² to 5.5 mg/cm², and especially ranges from 3 mg/cm² to 4.7 mg/cm².

Such a non-cyclic volatile oil is chosen in particular from non-cyclic volatile silicone oils.

The surface tension of a volatile oil is measured according to the protocol described below.

The non-cyclic volatile silicone oils are chosen from linear or branched volatile silicone oils.

The non-cyclic volatile silicone oil may be chosen from:
- the non-cyclic linear silicones of formula (I):

   R₃SiO-(R₂SiO)ₙ-SiR₃ (I)

   in which R, which may be identical or different, denotes:
- a saturated or unsaturated hydrocarbon-based radical containing from 1 to 10 carbon atoms and preferably from 1 to 6 carbon atoms, optionally substituted with one or more fluorine atoms or with one or more hydroxyl groups, or
- a hydroxyl group,
   one of the radicals R possibly being a phenyl group,
   n is an integer ranging from 0 to 8, preferably ranging from 2 to 6 and better still ranging from 3 to 5,
   the silicone compound of formula (I) containing not more than 18 carbon atoms;
- the branched silicones of formula (II) or (III) below:

   R₃SiO-[(R₃SiO)RSiO]-(R₂SiO)ₓ-SiR₃ (II)

   [R₃SiO]₄Si (III)

   in which R, which may be identical or different, denotes:
   - a saturated or unsaturated hydrocarbon-based radical containing from 1 to 10 carbon atoms, optionally substituted with one or more fluorine atoms or with one or more hydroxyl groups, or
   - a hydroxyl group,
   one of the radicals R possibly being a phenyl group,
   x is an integer from 0 to 8,
   the silicone compound of formula (II) or (III) containing not more than 18 carbon atoms.

Preferably, for the compounds of formulae (I), (II) and (III), the ratio between the number of carbon atoms and the number of silicone atoms is between 2.25 and 4.33. Advantageously, the compounds of formulae (I), (II) or (III) contain not more than 17 carbon atoms, preferably not more than 16 carbon atoms, and most preferably not more than 15 carbon atoms.

The silicones of formulae (I) to (III) may be prepared according to the known processes for synthesizing silicone compounds.

The volatile silicone is used as a mixture with another volatile silicone such that the mixture of volatile silicones has the said evaporation profile. The composition according to the invention comprises a mixture of volatile linear silicones, at least a mixture of the two volatile linear silicones dodecamethylpentasiloxane and decamethyltetrasiloxane.

Examples of non-cyclic volatile silicones are given below .

Among the silicones of formula (I) that may be mentioned are:
a) the following disiloxanes:
   hexamethyldisiloxane (surface tension = 15.9 mN/m), sold especially under the name DC 200 Fluid 0.65 cst by the company Dow Corning
   1,3-di-tert-butyl-1,1,3,3-tetramethyldisiloxane;
   1,3-dipropyl-1,1,3,3-tetramethyldisiloxane; heptylpentamethyldisiloxane;
   1,1,1-triethyl-3,3,3-trimethyldisiloxane; hexaethyldisiloxane;
   1,1,3,3-tetramethyl-1,3-bis(2-methylpropyl)disiloxane; pentamethyloctyldisiloxane;
   1,1,1-trimethyl-3,3,3-tris(1-methylethyl)disiloxane;
   1-butyl-3-ethyl-1,1,3-trimethyl-3-propyldisiloxane: pentamethylpentyldisiloxane;
   1-butyl-1,1,3,3-tetramethyl-3-(1-methylethyl)disiloxane;
   1,1,3,3-tetramethyl-1,3-bis(1-methylpropyl)disiloxane;
   1,1,3-triethyl-1,3,3-tripropyldisiloxane;
   (3,3-dimethylbutyl)pentamethyldisiloxane;
   (3-methylbutyl)pentamethyldisiloxane;
   (3-methylpentyl)pentamethyldisiloxane;
   1,1,1-triethyl-3,3-dimethyl-3-propyldisiloxane;
   1-(1,1-dimethylethyl)-1,1,3,3,3-pentamethyldisiloxane;
   1,1,1-trimethyl-3,3,3-tripropyldisiloxane:
   1,3-dimethyl-1,1,3,3-tetrakis(1-methylethyl)disiloxane;
   1,1-dibutyl-1,3,3,3-tetramethyldisiloxane:
   1,1,3,3-tetramethyl-1,3-bis(1-methylethyl)disiloxane;
   1,1,1,3-tetramethyl-3,3-bis(1-methylethyl)disiloxane;
   1,1,1,3-tetramethyl-3,3-dipropyldisiloxane;
   1,1,3,3-tetramethyl-1,3-bis(3-methylbutyl)disiloxane;
   butylpentamethyldisiloxane;
   pentaethylmethyldisiloxane;
   1,1,3,3-tetramethyl-1,3-dipentyldisiloxane;
   1,3-dimethyl-1,1,3,3-tetrapropyldisiloxane;
   1,1,1,3-tetraethyl-3,3-dimethyldisiloxane;
   1,1,1-triethyl-3,3,3-tripropyldisiloxane;
   1,3-dibutyl-1,1,3,3-tetramethyldisiloxane;
   hexylpentamethyldisiloxane;
b) the following trisiloxanes:
   octamethyltrisiloxane (surface tension = 17.4 mN/m), sold especially under the name DC 200 Fluid 1 cst by the company Dow Corning
   3-pentyl-1,1,1,3,5,5,5-heptamethyltrisiloxane;
   1-hexyl-1,1,3,3,5,5,5-heptamethyltrisiloxane;
   1,1,1,3,3,5,5-heptamethyl-5-octyltrisiloxane;
   1,1,1,3,5,5,5-heptamethyl-3-octyltrisiloxane, sold especially under the name "Silsoft 034" by the company OSI;
   1,1,1,3,5,5,5-heptamethyl-3-hexyltrisiloxane (surface tension = 20.5 mN/m), sold especially under the name "DC 2-1731" by the company Dow Corning;
   1,1,3,3,5,5-hexamethyl-1,5-dipropyltrisiloxane;
   3-(1-ethylbutyl)-1,1,1,3,5,5,5-heptamethyltrisiloxane;
   1,1,1,3,5,5,5-heptamethyl-3-(1-methylpentyl)trisiloxane;
   1,5-diethyl-1,1,3,3,5,5-hexamethyltrisiloxane;
   1,1,1,3,5,5,5-heptamethyl-3-(1-methylpropyl)trisiloxane;
   3-(1,1-dimethylethyl)-1,1,1,3,5,5,5-heptamethyltrisiloxane;
   1,1,1,5,5,5-hexamethyl-3,3-bis(1-methylethyl)trisiloxane;
   1,1,1,3,3,5,5-hexamethyl-1,5-bis(1-methylpropyl)trisiloxane;
   1,5-bis(1,1-dimethylethyl)-1,1,3,3,5,5-hexamethyltrisiloxane;
   3-(3,3-dimethylbutyl)-1,1,1,3,5,5,5-heptamethyltrisiloxane;
   1,1,1,3,5,5,5-heptamethyl-3-(3-methylbutyl)trisiloxane;
   1,1,1,3,5,5,5-heptamethyl-3-(3-methylpentyl)trisiloxane;
   1,1,1,3,5,5,5-heptamethyl-3-(2-methylpropyl)trisiloxane;
   1-butyl-1,1,3,3,5,5,5-heptamethyltrisiloxane;
   1,1,1,3,5,5,5-heptamethyl-3-propyltrisiloxane;
   3-isohexyl-1,1,1,3,5,5,5-heptamethyltrisiloxane;
   1,3,5-triethyl-1,1,3,5,5-pentamethyltrisiloxane;
   3-butyl-1,1,1,3,5,5,5-heptamethyltrisiloxane;
   3-tert-pentyl-1,1,1,3,5,5,5-heptamethyltrisiloxane;
   1,1,1,5,5,5-hexamethyl-3,3-dipropyltrisiloxane;
   3,3-diethyl-1,1,1,5,5,5-hexamethyltrisiloxane;
   1,5-dibutyl-1,1,3,3,5,5-hexamethyltrisiloxane;
   1,1,1,5,5,5-hexaethyl-3,3-dimethyltrisiloxane;
   3,3-dibutyl-1,1,1,5,5,5-hexamethyltrisiloxane;
   3-ethyl-1,1,1,3,5,5,5-heptamethyltrisiloxane;
   3-heptyl-1,1,1,3,5,5,5-heptamethyltrisiloxane;
   1-ethyl-1,1,3,3,5,5,5-heptamethyltrisiloxane;
c) the following tetrasiloxanes:
   decamethyltetrasiloxane (surface tension = 18 mN/m), sold especially under the name DC 200 Fluid 1.5 cst by the company Dow Corning;
   1,1,3,3,5,5,7,7-octamethyl-1,7-dipropyltetrasiloxane;
   1,1,1,3,3,5,7,7,7-nonamethyl-5-(1-methylethyl)tetrasiloxane;
   1-butyl-1,1,3,3,5,5,7,7,7-nonamethyltetrasiloxane;
   3,5-diethyl-1,1,1,3,5,7,7,7-octamethyltetrasiloxane;
   1,3,5,7-tetraethyl-1,1,3,5,7,7-hexamethyltetrasiloxane;
   3,3,5,5-tetraethyl-1,1,1,7,7,7-hexamethyltetrasiloxane;
   1,1,1,3,3,5,5,7,7-nonamethyl-7-phenyltetrasiloxane;
   3,3-diethyl-1,1,1,5,5,7,7,7-octamethyltetrasiloxane;
   1,1,1,3,3,5,7,7,7-nonamethyl-5-phenyltetrasiloxane;
d) the following pentasiloxanes:
   dodecamethylpentasiloxane (surface tension = 18.7 mN/m), sold especially under the name DC 200 Fluid 2 cst by the company Dow Corning;
   1,1,3,3,5,5,7,7,9,9-decamethyl-1,9-dipropylpentasiloxane;
   3,3,5,5,7,7-hexaethyl-1,1,1,9,9,9-hexamethylpentasiloxane;
   1,1,1,3,3,5,7,7,9,9,9-undecamethyl-5-phenylpentasiloxane;
   1-butyl-1,1,3,3,5,5,7,7,9,9,9-undecamethylpentasiloxane;
   3,3-diethyl-1,1,1,5,5,7,7,9,9,9-decamethylpentasiloxane;
   1,3,5,7,9-pentaethyl-1,1,3,5,7,9,9-heptamethylpentasiloxane;
   3,5,7-triethyl-1,1,1,3,5,7,9,9,9-nonamethylpentasiloxane;
   1,1,1-triethyl-3,3,5,5,7,7,9,9,9-nonamethylpentasiloxane;
e) the following hexasiloxanes:
   1-butyl-1,1,3,3,5,5,7,7,9,9,11,11,11-tridecamethylhexasiloxane;
   3,5,7,9-tetraethyl-1,1,1,3,5,7,9,11,11,11-decamethylhexasiloxane;
   tetradecamethylhexasiloxane;
f) hexadecamethylheptasiloxane;
g) octadecamethyloctasiloxane.

Among the silicones of formula (II) that may be mentioned are:
a) the following tetrasiloxanes:
   2-[3,3,3-trimethyl-1,1-bis[(trimethylsilyl)oxy]disiloxanyl]ethyl;
   1,1,1,5,5,5-hexamethyl-3-(2-methylpropyl)-3-[(trimethylsilyl)oxy]trisiloxane;
   3-(1,1-dimethylethyl)-1,1,1,5,5,5-hexamethyl-3-[(trimethylsilyl)oxy]trisiloxane;
   3-butyl-1,1,1,5,5,5-hexamethyl-3-[(trimethylsilyl)oxy]-trisiloxane;
   1,1,1,5,5,5-hexamethyl-3-propyl-3-[(trimethylsilyl)-oxy]trisiloxane;
   3-ethyl-1,1,1,5,5,5-hexamethyl-3-[(trimethylsilyl)-oxy]trisiloxane;
   1,1,1-triethyl-3,5,5,5-tetramethyl-3-(trimethylsiloxy)-trisiloxane;
   3-methyl-1,1,1,5,5,5-hexamethyl-3-[trimethylsilyl)oxy]-trisiloxane;
   3-[(dimethylphenylsilyl)oxy]-1,1,1,3,5,5,5-heptamethyltrisiloxane;
   1,1,1,5,5,5-hexamethyl-3-(2-methylpentyl)-3-[(trimethylsilyl)oxy]trisiloxane;
   1,1,1,5,5,5-hexamethyl-3-(4-methylpentyl)-3-[(trimethylsilyl)oxy]trisiloxane;
   3-hexyl-1,1,1,5,5,5-hexamethyl-3-[(trimethylsilyl)oxy]-trisiloxane;
   1,1,1,3,5,5,5-heptamethyl-3-[(trimethylsilyl)oxy]trisiloxane;
b) the following pentasiloxanes:
   1,1,1,3,5,5,7,7,7-nonamethyl-3-(trimethylsiloxy)tetrasiloxane;
   1,1,1,3,3,7,7,7-octamethyl-5-phenyl-5-[(trimethylsilyl)oxy]tetrasiloxane;
c) the following heptasiloxanes:
   1,1,1,3,5,5,7,7,9,9,11,11,11-tridecamethyl-3-[(trimethylsilyl)oxy]hexasiloxane.

Among the silicones of formula (III) that may be mentioned is:
1,1,1,5,5,5-hexamethyl-3,3-bis(trimethylsiloxy)trisiloxane.

It is also possible to use other volatile silicone oils chosen from:
a) the following tetrasiloxanes:
   2,2,8,8-tetramethyl-5-[(pentamethyldisiloxanyl)methyl]-3,7-dioxa-2,8-disilanonane;
   2,2,5,8,8-pentamethyl-5-[(trimethylsilyl)methoxy]-4,6-dioxa-2,5,8-trisilanonane;
   1,3-dimethyl-1,3-bis[(trimethylsilyl)methyl]-1,3-disiloxanediol;
   3-ethyl-1,1,1,5,5,5-hexamethyl-3-[3-(trimethylsiloxy)-propyl]trisiloxane;
   1,1,1,5,5,5-hexamethyl-3-phenyl-3-[(trimethylsilyl)-oxy]trisiloxane (Dow 556 Fluid);
b) the following pentasiloxanes:
   2,2,7,7,9,9,11,11,16,16-decamethyl-3,8,10,15-tetraoxa-2,7,9,11,16-pentasilaheptadecane;
   tetrakis[(trimethylsilyl)methyl] of silicic acid ester;
c) the following hexasiloxanes:
   3,5-diethyl-1,1,1,7,7,7-hexamethyl-3,5-bis[(trimethylsilyl)oxy]tetrasiloxane;
   1,1,1,3,5,7,7,7-octamethyl-3,5-bis[(trimethylsilyl)-oxy]tetrasiloxane;
d) heptasiloxane:
   1,1,1,3,7,7,7-heptamethyl-3,5,5-tris[(trimethylsilyl)-oxy]tetrasiloxane;
e) the following octasiloxanes:
   1,1,1,3,5,5,9,9,9-nonamethyl-3,7,7-tris[(trimethylsilyl)oxy]pentasiloxane;
   1,1,1,3,5,7,9,9,9-nonamethyl-3,5,7-tris[(trimethylsilyl)oxy]pentasiloxane;
   1,1,1,7,7,7-hexamethyl-3,3,5,5-tetrakis[(trimethylsilyl)oxy]tetrasiloxane.

Preferred volatile silicone oils that may be used include:
decamethyltetrasiloxane;
dodecamethylpentasiloxane;
3-butyl-1,1,1,3,5,5,5-heptamethyltrisiloxane;
1,1,1,3,5,5,5-heptamethyl-3-hexyltrisiloxane;
and mixtures thereof.

The volatile fatty phase of the composition according to the invention comprises a mixture of dodecamethylpentasiloxane and decamethyltetrasiloxane, especially in a dodecamethylpentasiloxane/decamethyltetrasiloxane weight ratio ranging from from 5/95 to 90/10, preferably ranging from 20/80 to 90/10, preferably ranging from 30/70 to 90/10, preferably ranging from 45/55 to 85/15, preferably ranging from 50/50 to 80/20, preferably ranging from 55/45 to 80/20, preferably ranging from 60/40 to 80/20, preferably ranging from 60/40 to 75/25, and preferentially ranging from 60/40 to 70/30.

A subject of the disclosure is also a composition comprising, in a physiologically acceptable medium, a volatile silicone fatty phase comprising a mixture of dodecamethylpentasiloxane and decamethyltetrasiloxane in a dodecamethylpentasiloxane/decamethyltetrasiloxane weight ratio ranging from 5/95 to 90/10, preferably ranging from 20/80 to 90/10, preferably ranging from 30/70 to 90/10, preferably ranging from 45/55 to 85/15, preferably ranging from 50/50 to 80/20, preferably ranging from 55/45 to 80/20, preferably ranging from 60/40 to 80/20, preferably ranging from 60/40 to 75/25, and preferentially ranging from 60/40 to 70/30.

Advantageously, the volatile silicone fatty phase of the composition is formed essentially, or even solely, from the mixture of dodecamethylpentasiloxane and decamethyltetrasiloxane described above.

According to the disclosure, the volatile fatty phase can comprise a mixture of dodecamethylpentasiloxane and 3-butyl-1,1,1,3,5,5,5-heptamethyltrisiloxane, especially in a dodecamethylpentasiloxane/3-butyl-1,1,1,3,5,5,5-heptamethyltrisiloxane weight ratio ranging from 75/25 to 50/50, preferably ranging from 70/30 to 55/45 and better still ranging from 65/35 to 55/45.

A subject of the disclosure is thus also a composition comprising, in a physiologically acceptable medium, a volatile silicone fatty phase comprising a mixture of dodecamethylpentasiloxane and 3-butyl-1,1,1,3,5,5,5-heptamethyltrisiloxane in a dodecamethylpentasiloxane/3-butyl-1,1,1,3,5,5,5-heptamethyltrisiloxane weight ratio ranging from 75/25 to 50/50, preferably ranging from 70/30 to 55/45 and better still ranging from 65/35 to 55/45.

Advantageously, the volatile silicone fatty phase of the composition is formed essentially, or even solely, of the mixture of dodecamethylpentasiloxane and 3-butyl-1,1,1,3,5,5,5-heptamethyltrisiloxane described above.

According to the disclosure, the volatile fatty phase can comprise a mixture of decamethyltetrasiloxane and 1,1,1,3,5,5,5-heptamethyl-3-hexyltrisiloxane, especially in a decamethyltetrasiloxane/1,1,1,3,5,5,5-heptamethyl-3-hexyltrisiloxane weight ratio ranging from 25/75 to 45/55, preferably ranging from 30/70 to 40/60 and better still ranging from 35/65 to 40/60.

A subject of the disclosure is thus also a composition comprising, in a physiologically acceptable medium, a volatile silicone fatty phase comprising a mixture of decamethyltetrasiloxane and 1,1,1,3,5,5,5-heptamethyl-3-hexyltrisiloxane in a decamethyltetrasiloxane/1,1,1,3,5,5,5-heptamethyl-3-hexyltrisiloxane weight ratio ranging from 25/75 to 45/55, preferably ranging from 30/70 to 40/60 and better still ranging from 35/65 to 40/60.

Advantageously, the volatile silicone fatty phase of the composition is formed essentially, or even solely, of the mixture of decamethyltetrasiloxane and 1,1,1,3,5,5,5-heptamethyl-3-hexyltrisiloxane described above.

According to the disclosure, the volatile fatty phase can comprise a mixture of 3-hexyl-1,1,1,3,5,5,5-heptamethyltrisiloxane and 3-butyl-1,1,1,3,5,5,5-heptamethyltrisiloxane, especially in a 3-hexyl-1,1,1,3,5,5,5-heptamethyltrisiloxane/3-butyl-1,1,1,3,5,5,5-heptamethyltrisiloxane weight ratio ranging from 45/55 to 70/30, preferably ranging from 50/50 to 65/35 and better still ranging from 55/45 to 60/40.

A subject of the disclosure is thus also a composition comprising, in a physiologically acceptable medium, a volatile silicone fatty phase comprising a mixture of 3-hexyl-1,1,1,3,5,5,5-heptamethyltrisiloxane and 3-butyl-1,1,1,3,5,5,5-heptamethyltrisiloxane in a 3-hexyl-1,1,1,3,5,5,5-heptamethyltrisiloxane/3-butyl-1,1,1,3,5,5,5-heptamethyltrisiloxane weight ratio ranging from 45/55 to 70/30, preferably ranging from 50/50 to 65/35 and better still ranging from 55/45 to 60/40.

Advantageously, the volatile silicone fatty phase of the composition is formed essentially, or even solely, of the mixture of 3-hexyl-1,1,1,3,5,5,5-heptamethyltrisiloxane and 3-butyl-1,1,1,3,5,5,5-heptamethyltrisiloxane described above.

According to the invention, the volatile silicone fatty phase of the composition according to the invention contains from 0 to 5% by weight and preferably from 0 to 1% by weight of volatile cyclic silicone oil, relative to the total weight of the volatile silicone fatty phase.

The non-cyclic volatile silicone oil, alone or as a mixture, may be present in a content ranging from 1% to 80% by weight, preferably ranging from 1% to 65% by weight and preferentially ranging from 1% to 50% by weight, relative to the total weight of the composition.

The composition according to the invention may also comprise a volatile non-silicone oil, chosen especially from volatile hydrocarbon-based or fluoro oils.

The term "hydrocarbon-based oil" means an oil formed essentially, or even consisting, of carbon and hydrogen atoms, and optionally of oxygen and nitrogen atoms, and containing no silicon or fluorine atoms. It may contain alcohol, ester, ether, carboxylic acid, amine and/or amide groups.

The volatile hydrocarbon-based oil may be chosen from volatile hydrocarbon-based oils containing from 8 to 16 carbon atoms and mixtures thereof, and especially C₈-C₁₆ branched alkanes, for instance C₈-C₁₆ isoalkanes (also known as isoparaffins), isododecane, isodecane, isohexadecane and, for example, the oils sold under the trade names "Isopars" or "Permethyls", C₈-C₁₆ branched esters, for instance isohexyl neopentanoate, and mixtures thereof; isododecane is preferably used.

The non-silicone volatile oil may be present in a content ranging from 0.1% to 50% by weight, preferably ranging from 0.1% to 40% by weight and preferentially ranging from 0.1% to 30% by weight, relative to the total weight of the composition.

The composition according to the invention may also comprise at least one non-volatile oil.

Non-volatile oils that may be mentioned include hydrocarbon-based oils of mineral or synthetic origin, such as linear or branched hydrocarbons, for instance liquid paraffin or derivatives thereof, liquid petroleum jelly, polydecenes, hydrogenated polyisobutene such as Parleam sold by the company Nippon Oil Fats, and squalane of synthetic or plant origin; oils of animal origin, for instance mink oil, turtle oil or perhydrosqualene; hydrocarbon-based oils of plant origin with a high triglyceride content consisting of fatty acid esters of glycerol, the fatty acids of which may have varying chain lengths, these chains possibly being linear or branched, and saturated or unsaturated, especially fatty acid triglycerides especially of 4 to 22 carbon atoms, for instance heptanoic or octanoic acid triglyceride, and capric/caprylic acid triglyceride, or alternatively hydroxylated triglycerides, such as sweet almond oil, beauty-leaf oil, palm oil, grapeseed oil, sesame oil, arara oil, rapeseed oil, sunflower oil, cotton oil, apricot oil, castor oil, alfalfa oil, marrow oil, blackcurrant oil, macadamia oil, musk rose oil, hazelnut oil, avocado oil, jojoba oil, olive oil, cereal (maize, wheat, barley or rye) germ oil, or karite butter; fatty acid esters, in particular of 4 to 22 carbon atoms, and especially of octanoic acid, of heptanoic acid, of lanolic acid, of oleic acid, of lauric acid or of stearic acid, for instance propylene glycol dioctanoate, propylene glycol monoisostearate, polyglyceryl-2 diisostearate or neopentylglycol diheptanoate;
synthetic esters of formula R₁COOR₂ in which R₁ represents a linear or branched higher fatty acid residue containing from 7 to 40 carbon atoms and R₂ represents a branched hydrocarbon-based chain containing from 3 to 40 carbon atoms, for instance purcellin oil (cetostearyl octanoate), isononyl isononanoate, C₁₂ to C₁₅ alkyl benzoate, 2-ethylhexyl palmitate, 2-octyldodecyl stearate, 2-octyldodecyl erucate, isostearyl isostearate, 2-octyldodecyl benzoate, alkyl or polyalkyl octanoates, decanoates or ricinoleates, isopropyl myristate, isopropyl palmitate, butyl stearate, hexyl laurate, diisopropyl adipate, 2-ethylhexyl palmitate, 2-hexyldecyl laurate, 2-octyldecyl palmitate, 2-octyldodecyl myristate, 2-diethylhexyl succinate, diisostearyl malate or isodecyl neopentanoate; hydroxylated esters, for instance isostearyl lactate, octyl hydroxystearate, octyldodecyl hydroxystearate, diisostearyl malate, triisocetyl citrate, glyceryl triisostearate or diglyceryl triisostearate; diethylene glycol diisononanoate; pentaerythritol esters; esters of aromatic acids and of alcohols containing 4 to 22 carbon atoms, especially tridecyl trimellitate; C₈-C₂₆ higher fatty acids such as oleic acid, linoleic acid, linolenic acid or isostearic acid; C₈-C₂₆ higher fatty alcohols such as oleyl alcohol, linoleyl alcohol, linolenyl alcohol, isostearyl alcohol or octyldodecanol; synthetic esters containing at least 7 carbon atoms, silicone oils such as polydimethylsiloxanes (PDMS) that are liquid at room temperature, linear, and optionally phenylated, such as phenyltrimethicones, phenyltrimethylsiloxydiphenyl-siloxanes, diphenyl dimethicones, diphenylmethyldiphenyltrisiloxanes, liquid 2-phenylethyl trimethylsiloxysilicates, optionally substituted with aliphatic and/or aromatic groups, for instance alkyl, alkoxy or phenyl groups, which are pendent and/or at the end of a silicone chain, these groups containing from 2 to 24 carbon atoms and being optionally fluorinated, or with functional groups such as hydroxyl, thiol and/or amine groups; polysiloxanes modified with fatty acids, fatty alcohols or polyoxyalkylenes, for instance dimethicone copolyols or alkylmethicone copolyols; liquid fluorosilicones; and mixtures thereof.

The non-volatile oil may be present in a content ranging from 0.1% to 60% by weight, preferably ranging from 0.5% to 50% by weight and preferentially ranging from 1% to 40% by weight, relative to the total weight of the composition.

The composition according to the invention may comprise at least one aqueous phase containing water. The water may be a floral water such as cornflower water and/or a mineral water such as eau de Vittel, eau de Lucas or eau de La Roche Posay and/or a spring water.

The aqueous phase may also comprise organic solvents that are miscible with water (at 25°C), for instance primary alcohols such as ethanol and isopropanol, glycols such as glycerol, propylene glycol, butylene glycol, dipropylene glycol, diethylene glycol, glycol ethers, C₁ to C₉ alkyl ethers of mono-, di- or tripropylene glycol, or mono-, di- or triethylene glycol, and mixtures thereof.

The composition may be an anhydrous composition, i.e. a composition containing less than 2% by weight of water, or even less than 0.5% of water, especially water-free, the water not being added during the preparation of the composition but corresponding to the residual water provided by the ingredients mixed therein.

The composition may contain other common cosmetic or dermatological ingredients that may be chosen especially from polymers, especially film-forming polymers and fixing polymers; surfactants; hair conditioners; dyestuffs; nacreous agents; opacifiers; organic solvents; fragrances; thickeners; gelling agents; waxes; pasty products; hair dyes; silicone resins; silicone gums; preserving agents; antioxidants; cosmetic active agents; sunscreens; pH stabilizers; vitamins; moisturizers; antiperspirants; deodorants; self-tanning compounds; and mixtures thereof.

Needless to say, a person skilled in the art will take care to select this or these optional additional compound(s), and/or the amount thereof, such that the advantageous properties of the composition according to the invention are not, or are not substantially, adversely affected by the envisaged addition.

The composition may advantageously contain a dyestuff that may be chosen from the lipophilic dyes, hydrophilic dyes, pigments and nacres usually used in cosmetic or dermatological compositions, and mixtures thereof. This dyestuff is generally present in a proportion of from 0.01% to 40%, preferably from 1% to 35% and better still from 5% to 25%, relative to the total weight of the composition.

The composition according to the invention may be in liquid, pasty or solid form, or in the form of a mousse or a spray. It may be an emulsion or an anhydrous composition.

The composition according to the invention may be used for making up, caring for or cleansing human keratin materials such as the skin (of the face, the body, the scalp or the lips), mucous membranes (inner edge of the eyelids), the hair, the nails, the eyelashes or the eyebrows.

The composition thus finds a particular application as a care composition for the body or the face; a cleansing composition for the body or the face, such as a shower gel, a bath gel or a makeup remover; a makeup composition for the body or the face, such as a foundation, a lipstick, a lipcare product, a nail varnish, a nailcare product, a mascara or an eyeliner; a fragrancing composition; a hair composition such as a hair dye composition or a permanent-reshaping hair composition; an antisun composition; a deodorant composition; a haircare or hair cleansing composition, such as a shampoo, a rinse-out or leave-in conditioner, a rinse-out composition to be applied before or after dyeing, bleaching, permanent-waving or relaxing the hair or between the two steps of a permanent-waving or relaxing operation; a hair composition for holding the hairstyle, such as a styling lacquer, gel, mousse or spray.
According to a particular embodiment of the invention,when the composition contains a mixture of dodecamethylpentasiloxane and decamethyltetrasiloxane in a dodecamethylpentasiloxane/decamethyltetrasiloxane weight ratio equal to 50/50, advantageously the composition is not a make-up remover composition.

The processes for manufacturing the products according to the invention do not differ in any way from the processes conventionally used in cosmetics and are entirely familiar to those skilled in the art.

The invention is illustrated in greater detail by means of the examples described below.

### Protocol 1:

### Measurement of the rate of evaporation of an oil:

15 g of oil or of the mixture of oils to be tested are placed in a crystallizing dish (diameter: 7 cm) placed on a balance that is inside a chamber of about 0.3 m³ with a regulated temperature (25°C) and a regulated hygrometry (50% relative humidity). The liquid is allowed to evaporate freely, without stirring, while providing ventilation with a fan (Papst-Motoren, reference 8550 N, rotating at 2700 rpm) positioned vertically above the crystallizing dish containing the solvent, the vanes facing the crystallizing dish and being 20 cm from the bottom of the crystallizing dish. The mass of oil remaining in the crystallizing dish is measured at regular intervals. The evaporation rates are expressed in mg of oil evaporated per unit of surface area (cm²) and per unit of time (minutes).

### Protocol 2:

### Measurement of the surface tension of a volatile oil:

The surface tension is determined by the "hanging drop" method. It is measured at 25 ± 1°C using a Fibro DAT 1100 tensiometer, sold by the company Fibro (Sweden). A drop of volatile oil is formed using a syringe controlled by the tensiometer at the outlet of a Teflon cannula with an inside diameter of 200 µm and an outside diameter of 1.5 mm, held in a vertical position. The volume of the drop is between 1 and 10 microlitres and preferably between 5 and 10 microlitres. The hanging drop is observed using a camera integrated into the measuring device, and its shape is parametized by the software of the Fibro DAT 1100 tensiometer using a resolution method of the Laplace equation. The result is given in mN/m.

### Example 1:

A foundation in the form of a water-in-oil emulsion having the composition below was prepared:

| | | |
|---|---|---|
| Cetyldimethicone copolyol | | |
| (Abil EM 90 from the company Goldschmidt) | 3 | g |
| Isostearyl diglyceryl succinate | | |
| (Imwitor 780K from the company Condea) | 0.6 | g |
| Decamethyltetrasiloxane | | |
| (DC 200 Fluid 1.5 cst from Dow Corning) | 6.48 | g |
| Dodecamethylpentasiloxane | | |
| (DC 200 Fluid 2 cst from Dow Corning) | 12.02 | g |
| Isododecane | 7.1 | g |
| Mixture of pigments (hydrophobic iron | | |
| oxides and titanium oxides) | 10 | g |
| Bentone | 1.6 | g |
| Polyamide powder | | |
| (Nylon-12 from Dupont de Nemours) | 8 | g |
| Magnesium sulphate | 0.7 | g |
| Preserving agent | 0.45 | g |
| Fragrance | 0.5 | g |
| Water qs | 100 | g |

### Example 2:

An oil-in-water foundation having the composition below was prepared:

| | | |
|---|---|---|
| Decamethyltetrasiloxane | | |
| (DC 200 Fluid 1.5 cst from Dow Corning) | 3.3 | g |
| Dodecamethylpentasiloxane | | |
| (DC 200 Fluid 2 cst from Dow Corning) | 7.7 | g |
| Hydrogenated polyisobutene (Parleam, NOF | | |
| Corporation) | 5 | g |
| 2-Ethylhexyl palmitate | 11 | g |
| Glyceryl isostearate | 4 | g |
| Stearic acid | 2 | g |
| Triethanolamine | 1 | g |
| Polyamide powder | | |
| (Nylon-12 from Dupont de Nemours) | 5 | g |
| Mixture of pigments | | |
| (iron oxides and titanium oxides) | 10 | g |
| Carboxymethylcellulose | 0.2 | g |
| Propylene glycol | 5 | g |
| Glycerol | 2 | g |
| Fragrance | 0.5 | g |
| Preserving agents | 0.4 | g |
| Water qs | 100 | g |

### Comparative Example 1:

A lipstick having the composition below was prepared:

| | | |
|---|---|---|
| Polyethylene wax | | |
| (Performalene 655, New Phase Technologies) | 20 | g |
| Decamethyltetrasiloxane | | |
| (DC 200 Fluid 1.5 cst from Dow Corning) | 18.2 | g |
| Dodecamethylpentasiloxane | | |
| (DC 200 Fluid 2 cst from Dow Corning) | 4 | g |
| Cyclopentadimethylsiloxane | | |
| (DC 245 Fluid from Dow Corning) | 6 | g |
| Isododecane | 51.8 | g |
| DC Red No. 7 Calcium Lake (pigment) | 6 | g |

### Example 3:

A care cream having the composition below was prepared:

### Fatty phase:

| | | |
|---|---|---|
| Mixture of glyceryl monostearate and of | | |
| polyethylene glycol stearate 100 EO (50/50 | | |
| by weight) | | |
| (Arlacel 165 from the company ICI) | 2.5 | g |
| - Stearyl alcohol | 0.5 | g |
| - Stearic acid | 1 | g |
| - Hydrogenated polyisobutene (Parleam, NOF | | |
| Corporation) | 9 | g |
| - Decamethyltetrasiloxane | | |
| (DC 200 Fluid 1.5 cst from Dow Corning) | 2.1 | g |
| - Dodecamethylpentasiloxane | | |
| (DC 200 Fluid 2 cst from Dow Corning) | 2.1 | g |

### Aqueous phase

| | | |
|---|---|---|
| - Crosslinked polyacrylic acid (Carbopol | | |
| 980) | 1 | g |
| - Triethanolamine | 0.03 | g |
| - Preserving agent | 0.3 | g |
| - Water qs | 100 | g |

### Example 4:

A makeup remover having the composition below was prepared:

| | | |
|---|---|---|
| Isopropyl palmitate | 8 | g |
| Decamethyltetrasiloxane | | |
| (DC 200 Fluid 1.5 cst from Dow Corning) | 2.8 | g |
| Dodecamethylpentasiloxane | | |
| (DC 200 Fluid 2 cst from Dow Corning) | 5.2 | g |
| Stearyl alcohol | 8 | g |
| Sucrose stearate | 2 | g |
| Stearic acid | 0.3 | g |
| Sodium hydroxide | 0.06 | g |
| Glycerol | 5 | g |
| Carbopol | 0.2 | g |
| Water qs | 100 | g |

## Claims

1. Composition comprising, in a physiologically acceptable medium, a volatile silicone fatty phase comprising at least one non-cyclic volatile silicone oil and from 0 to 5 % by weight of cyclic volatile silicone oil, relative to the total weight of the volatile silicone fatty phase, the volatile silicone fatty phase having an evaporation profile such that the mass of volatile silicone oil evaporated after 30 minutes ranges from 2 mg/cm² to 9 mg/cm², the volatile silicone fatty phase comprises a mixture of dodecamethylpentasiloxane and decamethyltetrasiloxane,
wherein the evaporation profile is determined according to the following protocol:
15 g of oil or of the mixture of oils to be tested are placed in a crystallizing dish with a diameter of 7 cm and placed on a balance that is inside a chamber of about 0.3 m³ with a regulated temperature of 25°C and a regulated hygrometry of 50% relative humidity. The liquid is allowed to evaporate freely, without stirring, while providing ventilation with a fan from Papst-Motoren, reference 8550 N, rotating at 2700 rpm and positioned vertically above the crystallizing dish containing the solvent, the vanes facing the crystallizing dish and being 20 cm from the bottom of the crystallizing dish.

2. Composition according to Claim 1, **characterized in that** the volatile silicone fatty phase has an evaporation profile such that the mass of volatile silicone oil evaporated after 30 minutes ranges from 2 mg/cm² to 8 mg/cm² and preferably ranges from 2 mg/cm² to 7 mg/cm².

3. Composition according to Claim 1 or 2, **characterized in that** the volatile silicone fatty phase has an evaporation profile such that the mass of volatile silicone oil evaporated after 30 minutes ranges from 2.3 mg/cm² to 6 mg/cm², preferably ranges from 3 mg/cm² to 5.5 mg/cm² and preferentially ranges from 3 mg/cm² to 4.7 mg/cm².

4. Composition according to the claim 1, **characterized in that** the dodecamethylpentasiloxane and the decamethyltetrasiloxane are present in a dodecamethylpentasiloxane/decamethyltetrasiloxane weight ratio ranging from 5/95 to 90/10, preferably ranging from 20/80 to 90/10, preferably ranging from 30/70 to 90/10, preferably ranging from 45/55 to 85/15, preferably ranging from 50/50 to 80/20, preferably ranging from 55/45 to 80/20, preferably ranging from 60/40 to 80/20, preferably ranging from 60/40 to 75/25, and preferentially ranging from 60/40 to 70/30.

5. Composition according to any one of the preceding claims, **characterized in that** the volatile silicone fatty phase contains from 0 to 1% by weight of cyclic volatile silicone oil, relative to the total weight of the volatile silicone fatty phase.

6. Composition according to any one of the preceding claims, **characterized in that** the non-cyclic volatile silicone oil, alone or as a mixture, is present in a content ranging from 1% to 80% by weight, preferably ranging from 1% to 65% by weight and preferentially ranging from 1% to 50% by weight, relative to the total weight of the composition.

7. Composition according to any one of Claims 1 or 4, **characterized in that** the dodecamethylpentasiloxane and the decamethyltetrasiloxane are present in a content ranging from 1% to 80% by weight, preferably ranging from 1% to 65% by weight and preferentially ranging from 1% to 50% by weight, relative to the total weight of the composition.

8. Composition according to any one of the preceding claims, **characterized in that** it comprises a volatile non-silicone oil.

9. Composition according to the preceding claim, **characterized in that** the non-silicone volatile oil is a volatile hydrocarbon-based oil.

10. Composition according to the preceding claim, **characterized in that** the hydrocarbon-based volatile oil is chosen from isododecane, isodecane, isohexadecane and isohexyl neopentanoate, and mixtures thereof.

11. Composition according to any one of Claims 8 to 10, **characterized in that** the volatile non-silicone oil is present in a content ranging from 0.1% to 50% by weight, preferably ranging from 0.1% to 40% by weight and preferentially ranging from 0.1% to 30% by weight, relative to the total weight of the composition.

12. Composition according to any one of the preceding claims, **characterized in that** it comprises a non-volatile oil.

13. Composition according to the preceding claim, **characterized in that** the non-volatile oil is present in a content ranging from 0.1% to 60% by weight, preferably ranging from 0.5% to 50% by weight and preferentially ranging from 1% to 40% by weight, relative to the total weight of the composition.

14. Composition according to any one of the preceding claims, **characterized in that** it comprises a cosmetic or dermatological ingredient chosen from film-forming polymers and fixing polymers; surfactants; hair conditioners; dyestuffs; nacreous agents; opacifiers; organic solvents; fragrances; thickeners; gelling agents; waxes; pasty products; hair dyes; silicone resins; silicone gums; preserving agents; antioxidants; cosmetic active agents; sunscreens; pH stabilizers; vitamins; moisturizers; antiperspirants; deodorants; self-tanning compounds; and mixtures thereof.

15. Composition according to any one of the preceding claims, **characterized in that** it is in the form of a care composition for the body or the face; a cleansing composition for the body or the face, such as a shower gel, a bath gel or a makeup remover; a makeup composition for the body or the face, such as a foundation, a lipstick, a lipcare product, a nail varnish, a nailcare product, a mascara or an eyeliner; a fragrancing composition; a hair composition such as a hair dye composition or a permanent-reshaping hair composition; an antisun composition; antiperspirants; deodorants; a haircare or hair cleansing composition, such as a shampoo, a rinse-out or leave-in conditioner, a rinse-out composition to be applied before or after dyeing, bleaching, permanent-waving or relaxing the hair or between the two steps of a permanent-waving or relaxing operation; a hair composition for holding the hairstyle, such as a styling lacquer, gel, mousse or spray.

16. Cosmetic makeup process or non-therapeutic treatment process for keratin materials, comprising the application to the keratin materials of a composition according to any one of the preceding claims.

## Patentansprüche

1. Zusammensetzung, die in einem physiologisch unbedenklichen Medium Folgendes umfasst: eine flüchtige Silikon-Fettphase, umfassend mindestens ein nichtzyklisches flüchtiges Silikonöl und 0 bis 5 Gew.-% zyklisches flüchtiges Silikonöl in Bezug auf das Gesamtgewicht der flüchtigen Silikon-Fettphase, wobei die flüchtige Silikon-Fettphase solch ein Verdunstungsprofil aufweist, dass die nach 30 Minuten verdunstete flüchtige Silikonölmenge im Bereich von 2 mg/cm² bis 9 mg/cm² liegt, wobei die flüchtige Silikon-Fettphase eine Mischung aus Dodecamethylpentasiloxan und Decamethyltetrasiloxan umfasst, wobei das Verdunstungsprofil nach der folgenden Vorschrift bestimmt wird: 15 g Testöl oder Testölmischung werden in eine Kristallisierschale mit einem Durchmesser von 7 cm gegeben und auf eine Waage, die sich in einer Kammer von ca. 0,3 m³ mit einer geregelten Temperatur von 25°C und einer geregelten Hygrometrie von 50% relativer Feuchtigkeit befindet, gestellt. Die Flüssigkeit wird frei ohne Rühren verdunsten gelassen, während mit einem Ventilator der Fa. Papst-Motoren, Bezugsnummer 8550 N, der mit 2700 U/min rotiert und der senkrecht oberhalb der Kristallisierschale, die das Lösungsmittel enthält, angebracht ist, belüftet wird, wobei die Ventilatorflügel gegenüber der Kristallisierschale liegen und 20 cm vom Boden der Kristallisierschale beabstandet sind.

2. Zusammensetzung nach Anspruch 1, **dadurch gekennzeichnet, dass** die flüchtige Silikon-Fettphase solch ein Verdunstungsprofil aufweist, dass die nach 30 Minuten verdunstete flüchtige Silikonölmenge im Bereich von 2 mg/cm² bis 8 mg/cm² und vorzugsweise im Bereich von 2 mg/cm² bis 7 mg/cm² liegt.

3. Zusammensetzung nach Anspruch 1 oder 2, **dadurch gekennzeichnet, dass** die flüchtige Silikon-Fettphase solch ein Verdunstungsprofil aufweist, dass die nach 30 Minuten verdunstete flüchtige Silikonölmenge im Bereich von 2,3 mg/cm² bis 6 mg/cm², vorzugsweise im Bereich von 3 mg/cm² bis 5,5 mg/cm² und bevorzugt im Bereich von 3 mg/cm² bis 4,7 mg/cm² liegt.

4. Zusammensetzung nach Anspruch 1, **dadurch gekennzeichnet, dass** das Dodecamethylpentasiloxan und das Decamethyltetrasiloxan in einem Dodecamethylpentasiloxan/Decamethyltetrasiloxan-Gewichtsverhältnis im Bereich von 5/95 bis 90/10, vorzugsweise im Bereich von 20/80 bis 90/10, vorzugsweise im Bereich von 30/70 bis 90/10, vorzugsweise im Bereich von 45/55 bis 85/15, vorzugsweise im Bereich von 50/50 bis 80/20, vorzugsweise im Bereich von 55/45 bis 80/20, vorzugsweise im Bereich von 60/40 bis 80/20, vorzugsweise im Bereich von 60/40 bis 75/25 und bevorzugt im Bereich von 60/40 bis 70/30 liegen.

5. Zusammensetzung nach einem der vorhergehenden Ansprüche, **dadurch gekennzeichnet, dass** die flüchtige Silikon-Fettphase 0 bis 1 Gew.-% zyklisches flüchtiges Silikonöl in Bezug auf das Gesamtgewicht der flüchtigen Silikon-Fettphase enthält.

6. Zusammensetzung nach einem der vorhergehenden Ansprüche, **dadurch gekennzeichnet, dass** das nichtzyklische flüchtige Silikonöl, allein oder als Mischung, in einem Gehalt im Bereich von 1 Gew.-% bis 80 Gew.-%, vorzugsweise im Bereich von 1 Gew.-% bis 65 Gew.-% und bevorzugt im Bereich von 1 Gew.-% bis 50 Gew.-% in Bezug auf das Gesamtgewicht der Zusammensetzung vorliegt.

7. Zusammensetzung nach einem der Ansprüche 1 oder 4, **dadurch gekennzeichnet, dass** das Dodecamethylpentasiloxan und das Decamethyltetrasiloxan in einem Gehalt im Bereich von 1 Gew.-% bis 80 Gew.-%, vorzugsweise im Bereich von 1 Gew.-% bis 65 Gew.-% und bevorzugt im Bereich von 1 Gew.-% bis 50 Gew.-% in Bezug auf das Gesamtgewicht der Zusammensetzung vorliegt.

8. Zusammensetzung nach einem der vorhergehenden Ansprüche, **dadurch gekennzeichnet, dass** sie ein flüchtiges Nichtsilikonöl umfasst.

9. Zusammensetzung nach dem vorhergehenden Anspruch, **dadurch gekennzeichnet, dass** es sich bei dem flüchtigen Nichtsilikonöl um ein flüchtiges Öl auf Kohlenwasserstoffbasis handelt.

10. Zusammensetzung nach dem vorhergehenden Anspruch, **dadurch gekennzeichnet, dass** das flüchtige Öl auf Kohlenwasserstoffbasis aus der Reihe Isododecan, Isodecan, Isohexadecan und Isohexylneopentanoat und deren Mischungen ausgewählt ist.

11. Zusammensetzung nach einem der Ansprüche 8 bis 10, **dadurch gekennzeichnet, dass** das flüchtige Nichtsilikonöl in einem Gehalt im Bereich von 0,1 Gew.-% bis 50 Gew.-%, vorzugsweise im Bereich von 0,1 Gew.-% bis 40 Gew.-% und bevorzugt im Bereich von 0,1 Gew.-% bis 30 Gew.-% in Bezug auf das Gesamtgewicht der Zusammensetzung vorliegt.

12. Zusammensetzung nach einem der vorhergehenden Ansprüche, **dadurch gekennzeichnet, dass** sie ein nichtflüchtiges Öl umfasst.

13. Zusammensetzung nach dem vorhergehenden Anspruch, **dadurch gekennzeichnet, dass** das nichtflüchtige Öl in einem Gehalt im Bereich von 0,1 Gew.-% bis 60 Gew.-%, vorzugsweise im Bereich von 0,5 Gew.-% bis 50 Gew.-% und bevorzugt im Bereich von 1 Gew.-% bis 40 Gew.-% in Bezug auf das Gesamtgewicht der Zusammensetzung vorliegt.

14. Zusammensetzung nach einem der vorhergehenden Ansprüche, **dadurch gekennzeichnet, dass** sie einen kosmetischen oder dermatologischen Bestandteil aus der Reihe filmbildende Polymere und fixierende Polymere, Tenside, Haarkonditionierungsmittel, Farbstoffe, Perlglanzmittel, Opakisierungsmittel, organische Lösungsmittel, Parfums, Verdickungsmittel, Geliermittel, Wachse, pastöse Produkte, Haarfarben, Silikonharze, Silikongummis, Konservierungsmittel, Antioxidantien, Kosmetikwirkstoffe, Sonnenschutzfilter, pH-Stabilisatoren, Vitamine, feuchtigkeitsspendende Mittel, Antiperspirantien, Deodorantien, Selbstbräunungsmittel und deren Mischungen umfasst.

15. Zusammensetzung nach einem der vorhergehenden Ansprüche, **dadurch gekennzeichnet, dass** sie in Form einer Pflegezusammensetzung für den Körper oder das Gesicht; einer Reinigungszusammensetzung für den Körper oder das Gesicht wie einem Duschgel, einem Badegel oder einem Makeup-Entferner; einer Makeup-Zusammensetzung für den Körper oder das Gesicht, wie einer Makeup-Grundierung, eines Lippenstifts, eines Lippenpflegeprodukts, eines Nagellacks, eines Nagelpflegeprodukts, einer Wimperntusche oder eines Eyeliners; einer duftspendenden Zusammensetzung; einer Haarzusammensetzung wie einer Haarfärbezusammensetzung oder einer Dauerwellen-Haarzusammensetzung; einer Sonnenschutzzusammensetzung, von Antiperspirantien; von Deodorantien; einer Haarpflege- oder Haarreinigungszusammensetzung wie eines Shampoos, eines gegebenenfalls auszuspülenden Konditionierungsmittels, einer auszuspülenden Zusammensetzung, die vor oder nach dem Färben, Bleichen, der Dauerwellbehandlung oder dem Entkrausen des Haars oder zwischen den zwei Schritten eines Dauerwellvorgangs oder Haarentkrausvorgangs aufzutragen ist; einer Haarzusammensetzung, die dazu dient, um der Frisur Halt zu geben, wie eines Haarlacks, Haargels, Haarschaums oder Haarsprays vorliegt.

16. Kosmetisches Makeup-Verfahren oder nichttherapeutisches Behandlungsverfahren für Keratinsubstanzen, bei dem man eine Zusammensetzung nach einem der vorhergehenden Ansprüche auf die Keratinsubstanzen aufträgt.

## Revendications

1. Composition comprenant, dans un milieu physiologiquement acceptable, une phase grasse de silicone volatile comprenant au moins une huile silicone volatile non cyclique et de 0 à 5 % en poids d'huile silicone volatile cyclique, par rapport au poids total de la phase grasse de silicone volatile ayant un profil d'évaporation de sorte que la masse d'huile silicone volatile évaporée après 30 minutes soit dans la plage de 2 mg/cm² à 9 mg/cm², la phase grasse de silicone volatile comprend un mélange de dodécaméthylpentasiloxane et de décaméthyltétrasiloxane, le profil d'évaporation étant déterminé selon le protocole suivant :
15 g d'huile ou du mélange d'huiles soumis à essai sont placés dans une capsule ayant un diamètre de 7 cm et placée sur une balance qui est à l'intérieur d'une chambre d'environ 0,3 m³ avec une température régulée de 25 °C et une hygrométrie régulée de 50 % d'humidité relative. On laisse le liquide s'évaporer librement, sans agitation, en effectuant une ventilation avec un ventilateur de Papst-Motoren, référence 8550 N, tournant à 2700 tours/min et positionné verticalement au-dessus de la capsule de cristallisation contenant le solvant, les pales faisant face à la capsule de cristallisation et étant à 20 cm du fond de la capsule de cristallisation.

2. Composition selon la revendication 1, **caractérisée en ce que** la phase grasse de silicone volatile a un profil d'évaporation de sorte que la masse d'huile silicone volatile évaporée après 30 minutes soit dans la plage de 2 mg/cm² à 8 mg/cm² et soit de préférence dans la plage de 2 mg/cm² à 7 mg/cm².

3. Composition selon la revendication 1 ou 2, **caractérisée en ce que** la phase grasse de silicone volatile a un profil d'évaporation de sorte que la masse d'huile silicone volatile évaporée après 30 minutes soit dans la plage de 2,3 mg/cm² à 6 mg/cm², de préférence dans la plage de 3 mg/cm² à 5,5 mg/cm² et est de préférence dans la plage de 3 mg/cm² à 4,7 mg/cm².

4. Composition selon la revendication 1, **caractérisé en ce que** le dodécaméthylpentasiloxane et le décaméthyltétrasiloxane sont présents dans un rapport en poids de dodécaméthylpentasiloxane / décaméthyltétrasiloxane sont dans la plage de 5/95 à 90/10, de préférence dans la plage de 20/80 à 90/10, de préférence dans la plage de 30/70 à 90/10, de préférence dans la plage de 45/55 à 85/15, de préférence dans la plage de 50/50 to 80/20, de préférence dans la plage de 55/45 to 80/20, de préférence dans la plage de 60/40 à 80/20, de préférence dans la plage de 60/40 à 75/25, et de préférence dans la plage de 60/40 à 70/30.

5. Composition selon l'une quelconque des revendications précédentes, **caractérisée en ce que** la phase grasse de silicone volatile contient de 0 à 1 % en poids d'huile silicone volatile cyclique, par rapport au poids total de la phase grasse de silicone volatile.

6. Composition selon l'une quelconque des revendications précédentes, **caractérisée en ce que** l'huile silicone volatile non cyclique, seule ou en mélange, est présente à une teneur dans la plage de 1 % à 80 % en poids, de préférence dans la plage de 1 % à 65 % en poids et de préférence dans la plage de 1 % à 50 % en poids, par rapport au poids total de la composition.

7. Composition selon l'une quelconque des revendications 1 ou 4, **caractérisée en ce que** le dodécaméthylpentasiloxane et le décaméthyltétrasiloxane sont présents à une teneur dans la plage de 1 % à 80 % en poids, de préférence dans la plage de 1 % à 65 % en poids et de préférence dans la plage de 1 % à 50 % en poids, par rapport au poids total de la composition.

8. Composition selon l'une quelconque des revendications précédentes, **caractérisée en ce qu'**elle comprend une huile non-silicone volatile.

9. Composition selon la revendication précédente, **caractérisée en ce que** l'huile non-silicone volatile est une huile volatile à base d'hydrocarbures.

10. Composition selon la revendication précédente, **caractérisée en ce que** l'huile volatile à base d'hydrocarbures est choisie parmi l'isododécane, l'isodécane, l'isohexadécane et le néopentanoate d'isohexyle et des mélanges de ceux-ci.

11. Composition selon l'une quelconque des revendications 8 à 10, **caractérisée en ce que** l'huile non-silicone volatile est présente à une teneur dans la plage de 0,1 % à 50 % en poids, de préférence dans la plage de 0,1 % à 40 % en poids et de préférence dans la plage de 0,1 % à 30 % en poids, par rapport au poids total de la composition.

12. Composition selon l'une quelconque des revendications précédentes, **caractérisée en ce qu'**elle comprend une huile non volatile.

13. Composition selon la revendication précédente, **caractérisée en ce que** l'huile non volatile est présente à une teneur dans la plage de 0,1 % à 60 % en poids, de préférence dans la plage de 0,5 % à 50 % en poids et de préférence dans la plage de 1 % à 40 % en poids, par rapport au poids total de la composition.

14. Composition selon l'une quelconque des revendications précédentes, **caractérisée en ce qu'**elle comprend un composant cosmétique ou dermatologique choisi parmi des polymères filmogènes et des polymères de fixation ; des tensioactifs ; des conditionneurs capillaires ; des colorants ; des agents nacrés ; des opacifiants ; des solvants organiques ; des parfums ; des épaississants ; des agents gélifiants ; des cires ; des produits pâteux ; des colorants capillaires ; des résines silicone ; des gommes silicone ; des agents conservateurs ; des antioxydants ; des agents actifs cosmétiques ; des écrans solaires ; des stabilisants de pH ; des vitamines ; des agents hydratants ; des anti-transpirants des déodorants ; des composés autobronzants ; et des mélanges de ceux-ci.

15. Composition selon l'une quelconque des revendications précédentes, **caractérisée en ce qu'**elle est sous la forme d'une composition de soins pour le corps ou le visage ; une composition nettoyante pour le corps ou le visage, tel qu'un gel douche, un gel de bain ou un démaquillant ; une composition de maquillage pour le corps ou le visage, tel qu'un fond de teint, un rouge à lèvres, un produit de soin des lèvres, un vernis à ongles, un produit de soin des ongles, un mascara ou un eyeliner ; une composition de parfum ; une composition capillaire telle qu'une composition de coloration des cheveux ou une composition capillaire de remodelage permanent des cheveux ; une composition antisolaire ; des anti-transpirants ; des déodorants ; une composition de soin capillaire ou de nettoyage capillaire, telle qu'un shampoing, un conditionneur avec ou sans rinçage, une composition à rincer à appliquer avant ou après une coloration, une décoloration, un frisage permanent ou un défrisage permanent des cheveux ou entre les deux étapes d'une opération de frisage ou de défrisage permanent ; une composition capillaire pour maintenir une coiffure, telle qu'une laque, un gel, une mousse ou un aérosol capillaire.

16. Procédé de maquillage cosmétique ou procédé de traitement non thérapeutique pour des matériaux de kératine, comprenant l'application sur les matériaux de kératine d'une composition selon l'une quelconque des revendications précédentes.
